Europäisches Patentamt

European Patent Office

Office européen des brevets

(11) Veröffentlichungsnummer: **0 642 790 A2**

# EUROPÄISCHE PATENTANMELDUNG

(21) Anmeldenummer: 94112949.6

(51) Int. Cl.⁶: **A61K 31/19**, C07C 59/265

(22) Anmeldetag: 19.08.94

(30) Priorität: **25.08.93 DE 4328577**

(43) Veröffentlichungstag der Anmeldung:
**15.03.95 Patentblatt 95/11**

(84) Benannte Vertragsstaaten:
**AT BE CH DE DK ES FR GB GR IE IT LI LU MC NL PT SE**

(71) Anmelder: **MADAUS AG**
**Ostmerheimer Strasse 198**
**D-51109 Köln (DE)**

(72) Erfinder: **Schwille, Paul-Otto Prof. Dr. Dr.**
**Finkenweg 5**
**D-91080 Uttenreuth (Erlangen) (DE)**
Erfinder: **Schick, Christoph Dr. med.**
**Friedrich-Rückert-Strasse 2**
**D-90419 Nürnberg (DE)**

(54) Alkalicitrate und deren Verwendung als Arzneimittel, insbesondere gegen Arteriosklerose.

(57) Die Erfindung betrifft die Verwendung von Alkalimetallhydrogencitrat-Zusammensetzungen oder -Verbindungen bei der Prophylaxe und Auflösung von Kalkablagerungen in Blutgefäßen, insbesondere bei Arteriosklerose.

Gegenstand der Erfindung ist die Verwendung von Alkalimetallhydrogencitraten als Arzneimittel.

Es ist bekannt, daß ein saures Alkalicitrat der Formel

$$K_6 Na_6 H_3 (C_6 H_5 O_7)_5$$

als Hydrat mit 1 bis 4 Wassermolekülen zur Auflösung von Harnsäuresteinen und zur Verhinderung ihrer erneuten Bildung verabreicht wird. So beschreibt die DE PS 27 27 304 die Verwendung von diesem sauren Alkalicitrat zur Bekämpfung der Urolithiasis, wobei bei der Anwendung als Arzneimittel Citrat-, Natrium- und Kalium-Ionen in einem bestimmten äquivalenten Verhältnis freigesetzt werden und eine therapeutisch gewünschte pH-Wert-Erhöhung des Harns auf pH 6,2 bis 7,0 bewirkt wird. Bei Patienten mit Hyperurikusurie bewirkt saures Alkalicitrat, welches im Zustand eines definierten Kristallisats vorliegt, durch die gezielte pH-Anhebung im Harn eine Auflösung und zudem eine Verhinderung der Neubildung von Harnsäuresteinen.

Der Stand der Technik beschreibt zwar die Anwendung von Alkalicitrat und Citronensäure als Arzneimittel bei der Behandlung der oben genannten Krankheiten, dabei beschränken sich jedoch die bisher bekannten Therapien mit den o.g. Alkalicitraten ausschließlich auf Erkrankungen und Schädigungen der ableitenden Harnwege.

Die Arteriosklerose stellt Blutgefäßerkrankungen dar, bei denen chronisch fortschreitende, herdförmige Ablagerungen oder Plaques in der Media der Gefäße auftreten, die zu einer Verringerung der Elastizität, einer gesteigerten Verhärtung und Verdickung der Gefäßwände führen. Diese Ablagerungen bestehen aus Fetteinlagerungen und/oder Kalkeinlagerungen. Bei der krankhaften Kalkeinlagerung (= Calcinosis) werden Calciumverbindungen, vorwiegend sind es Calciumphosphate, als Vorstufen des Hydroxylapatits eingelagert. Sowohl bei der Verkalkung (Mineralisierung oder Calcifizierung) im Knochen als auch bei der Verkalkung in den Blutgefäßen (Arteriosklerose oder volkstümlich "Arterienverkalkung" genannt) von Hirn, Herz und Nieren zeigt das Auftreten von kristallin vorliegendem Hydroxylapatit das Endstadium der Mineralisation oder Verkalkung an.

Der Stand der Technik schlägt zur Therapie der Arteriosklerose die Verwendung einer Reihe von Medikamenten vor, die jedoch aufgrund ihrer Wirkung auf den Cholesterinspiegel vorwiegend die Fett- und nicht die Kalkeinlagerungen in den Gefäßen zu verhindern helfen und eine Vielzahl unerwünschter Nebenwirkungen hervorrufen.

So wird die Anwendung von Clofibrinsäurederivaten beschrieben, welche aber bereits nach kurzfristiger Einnahme Durchfall, Erbrechen, Hautausschlag und Haarausfall beim Patienten hervorrufen. Ganz abgesehen davon, daß, da Clofibrinsäuren u.U. Antikoagulantieneffekte verstärken, eine Behandlung mit Clofibrinsäuren eine dauernde und intensive Kontrolle der Prothrombinzeit bei Patienten erforderlich macht.

Auch die Applizierung von Nicotinsäureverbindungen führt nicht zu einer zufriedenstellenden Therapie der arteriosklerotisch hervorgerufenen Ablagerungsbildungen, da Kontraindikationen wie Gicht, Lebererkrankungen und akute Blutungen zu beachten sind. Weil oft die arteriosklerotisch hervorgerufene Ablagerungsbildung mit Diabeteserkrankungen (Diabetes mellitus) einhergeht, stellt die Verwendung von Nicotinsäuren wegen der durch diese Medikamente hervorgerufenen Änderung in der Glukosetoleranz keine erfolgversprechende Therapie dar.

Ebenso führt die vorgeschlagene Verwendung von Ionenaustauschern wegen der schlechten Verträglichkeit aufgrund von Nierensteinbildung und weiterer Nebenwirkungen wie Bindung von lebenswichtigen fettlöslichen Vitaminen an die Ionenaustauschermatrix im menschlichen Organismus nach Einnahme zu Avitaminosen.

Da wegen der unzureichenden Therapiemöglichkeiten der Ablagerungsbildung bei arteriosklerotischen Erkrankungen insbesondere bez. der gezielten Therapie gegen kalkhaltige Ablagerungsbildungen bisher keine erfolgversprechenden Medikamente zu Verfügung stehen, wird ein "Therapieerfolg folglich gerade auf diesem Gebiet als fraglich" angesehen (Lehrbuch der Pharmakologie und Toxikologie, VCH Verlag, Weinheim 1982, Seite 425) und vielmehr als letzter Ausweg "eine maßvolle Ernährung vorgeschlagen" (Lehrbuch, Innere Medizin, Springer Verlag, Heidelberg 1982, Seite 600).

Die Aufgabe der vorliegenden Erfindung besteht nun darin, ein Mittel zur Auflösung von Ablagerungen bei der Behandlung von Arteriosklerose anzugeben, das sowohl für den menschlichen Organismus gut verträglich ist, i.e. eine komplikationslose Verstoffwechslung im menschlichen Organismus aufweist, als auch bei der Einnahme weiterer Medikamente keine Wechselwirkungen zeigt. Da man heutzutage aufgrund des wachsenden Kostendrucks in der Therapeutik außerdem bestrebt ist, statt eine bereits manifeste Krankheit zu therapieren, einer zukünftigen Erkrankung vorzubeugen, sollte das Medikament das Entstehen der Ablagerungen wie Kalkeinlagerungen in der Media der Blutgefäße nicht nur abbauen helfen, sondern auch möglichst frühzeitig das Einlagern der Calciumphosphate als Vorstufen des Hydroxylapatits verhindern.

Dazu ist ein Arzneimittel erforderlich, welches bei einer Langzeiteinnahme keine oder nur vernachlässigbare Nebenwirkungen aufweist und somit den Stoffwechsel und die Nierentätigkeit nicht beeinflußt; zumal zu berücksichtigen ist, daß bei einer vorbeugenden Behandlung die Nieren gerade aufgrund der dauernden Medikamenteneinnahme von Nebenwirkungen in erster Linie betroffen sein werden. Darüber hinaus ist zu beachten, daß das Medikament auch von Diabetikern eingenommen werden kann, weil gerade Arteriosklerose und Diabetes mellitus oft im Alter gleichzeitig auftreten.

Ebenso ist es angebracht, die Wirksubstanz so zu wählen, daß sie preiswert herstellbar ist, um die Herstellungskosten des Medikaments zu senken und eine ubiquitäre Verwendung als Heilmittel zu gewährleisten.

Überraschenderweise wurde nun gefunden, daß die im Anspruch 1 definierten Zusammensetzungen und Verbindungen diese Aufgabe lösen.

Gegenstand der Erfindung ist daher die Verwendung mindestens einer Alkalimetallhydrogencitratzusammensetzung oder -Verbindung oder eines Hydrats davon mit der Zusammensetzung Kaliumnatriumhydrogencitrat im Molverhältnis

$K:Na:H:(C_6H_5O_7)$ w:x:y:z

worin w für eine ganze Zahl von 0 bis 15; x für eine ganze Zahl von 0 bis 15; y für eine ganze Zahl von 0 bis 3; z für eine ganze Zahl von 1 bis 5 stehen; wobei
w + x + y = 3; 6; 9; 12 oder 15 bei entsprechend
z = 1; 2; 3; 4 oder 5 und w + x = 1 bis 15 sind, zur Prophylaxe und Auflösung von Kalkablagerungen in Blutgefäßen.

Die Kalium-Natrium-Hydrogencitrate kommen insbesondere als Hydrat bzw. Hydratgemisch zur Anwendung. Im allgemeinen sind 1 bis 4 Wassermoleküle enthalten.

Die Erfindung betrifft eine neue, einheitliche Verbindung, nämlich

$KNaH(C_6H_5O_7)$

bzw. ein Hydrat, davon insbesondere mit 1 bis 4 Wassermolekülen. Das Verfahren zur Herstellung dieser Verbindung ist dadurch gekennzeichnet daß man entweder

a) Trinatriumcitrat, Trikaliumcitrat und Citronensäure im Molverhältnis 1:1:1 gemeinsam in der 3 bis 5fachen Gewichtsmenge in siedendem demineralisiertem Wasser, bezogen auf das Gewicht der Citronensäure, auflöst, die Temperatur der Lösung 60 °C nicht unterschreiten läßt oder

b) Citronensäure unter Rühren in der 0,5 bis 1fachen Gewichtsmenge deminieralisiertem Wasser, bezogen auf das Gewicht der eingesetzten Citronensäure, bei 90 °C löst anschließend Natriumcarbonat sowie Kaliumcarbonat in solchen Mengen zufügt, daß sich ein Molverhältnis von Natriumcarbonat:Kaliumcarbonat:Citronensäure von 1:1:2 ergibt oder

c) Citronensäure unter Rühren in der 0,5 bis 1fachen Gewichtsmenge demineralisiertem Wasser, bezogen auf das Gewicht der eingesetzten Citronensäure, bei 90 °C löst, anschließend Natriumhydroxid bzw. Natriumbicarbonat sowie Kaliumhydroxid bzw. Kaliumbicarbonat in solchen Mengen zufügt, daß sich ein Molverhältnis von Natriumhydroxid bzw. Natriumbicarbonat:Kaliumhydroxid bzw. Kaliumbicarbonat:Citronensäure von 1:1:1 ergibt

und jeweils die homogene heiße Lösung einer Schnelltrocknung unterwirft.

Die Erfindung betrifft auch ein pharmazeutisches Mittel, das diese Verbindung ggf. in Kombination mit üblichen Trägern und/oder Zusatzstoffen enthält.

Die Herstellung der erfindungsgemäß zur Anwendung kommenden Zusammensetzungen bzw. Verbindungen ist dem Fachmann aus der DE PS 27 27 304 bekannt. Darin wird die Herstellung der Verbindung $K_6Na_6H_3(C_6H_5O_7)_5$ beschrieben. Die Verfahrensvorschriften der DE PS 27 27 304 werden bez. der molaren Verhältnisse der Ausgangstoffe zueinander entsprechend den angegebenen Verhältnissen der Alkalimetalle zum Citrat verändert.

**Herstellungsbeispiel 1**: $K_2H(C_6H_5O_7)_5$ als Hydratgemisch

Die Herstellung von $K_2H(C_6H_5O_7)$ erfolgt, indem man 960,6 kg Citronensäure unter Rühren in der 0.5 bis 1fachen Gewichtsmenge demineralisiertem Wasser, bezogen auf das Gewicht der eingesetzten Citronensäure, bei 90 °C löst, anschließend 1000 kg wasserfreies Kaliumbicarbonat zufügt und jeweils die homogene heiße Lösung der Schnelltrocknung unterwirft.

3

Anstatt Kaliumbicarbonat kann man 690 kg wasserfreies Kaliumcarbonat oder 560 kg wasserfreies Kaliumhydroxid einsetzen.

Anschließend wird unter Beachtung, daß kein Bodenkörper auftritt, die homogene Lösung mittels einer Pumpe kontinuierlich auf einen Zweiwalzentrockner überführt und schnell zur Trockne gebracht. Die Schichtdicke auf den Walzen beträgt hierbei 0.5 bis 0,8 mm. Die Trockenwalzen werden innen mit Sattdampf von 5 bis 7 bar Überdruck beaufschlagt, so daß eine Walzenoberflächentemperatur von 140°C bis 160°C resultiert. Durch Drehzahleinstellung wird eine Verweilzeit des Gutes auf der Walze von ca. 5 Sekunden erreicht. Die Leistung beträgt 30 bis 35 kg Trockengut pro m$^2$ Heizfläche und Stunde. Die Zylinder der Walzen bestehen aus feinkörnigem Spezialgrauguß mit perlitischem Gefüge, sie sind außen und innen gedreht, geschliffen und stark hartverchromt.

Die Resttrocknung erfolgt auf Tellertrocknern auf ca. 3% $H_2O$. Das Gewicht des entstandenen Endprodukts, berechnet als wasserfreie Substanz, beträgt 1.340 kg.

**Herstellungsbeispiel 2**: $K_3(C_6H_5O_7)$ als Hydratgemisch

Die Herstellung von $K_3(C_6H_5O_7)$ erfolgt, indem man 960,6 kg Citronensäure unter Rühren in der 0.5 bis 1fachen Gewichtsmenge demineralisiertem Wasser, bezogen auf das Gewicht der eingesetzten Citronensäure, bei 90°C löst, anschließend 1500 kg wasserfreies Kaliumbicarbonat zufügt und jeweils die homogene heiße Lösung der Schnelltrocknung, wie in Herstellungsbeispiel 1 angegeben, unterwirft. Das Gewicht des Endprodukts, berechnet als wasserfreie Substanz, beträgt 1.530 kg.

Anstatt Kaliumbicarbonat, wie beschrieben, kann man 1035 kg wasserfreies Kaliumcarbonat oder 840 kg wasserfreies Kaliumhydroxid einsetzen.

**Herstellungsbeispiel 3**: $Na_3(C_6H_5O_7)$ als Hydratgemisch

Die Herstellung von $Na_3(C_6H_5O_7)$ erfolgt, indem man 960,6 kg Citronensäure unter Rühren in der 0,5 bis 1fachen Gewichtsmenge demineralisiertem Wasser, bezogen auf das Gewicht der eingesetzten Citronensäure, bei 90°C löst, anschließend 1260 kg Natriumbicarbonat zufügt und jeweils die homogene heiße Lösung der Schnelltrocknung, wie in Herstellungsbeispiel 1 angegeben, unterwirft. Das Gewicht des Endprodukts, berechnet als wasserfreie Substanz, beträgt 1290 kg.

Anstatt Natriumbicarbonat, wie beschrieben, kann man 945 kg wasserfreies Natriumcarbonat oder 600 kg wasserfreies Natriumhydroxid einsetzen.

**Herstellungsbeispiel 4**: $KNaH(C_6H_5O_7)$ als Hydratgemisch

Die Herstellung von $KNaH(C_6H_5O_7)$ erfolgt, indem man 230,4 kg Citronensäure, 388,8 kg Trikaliumcitrat . 1$H_2O$ und 352,8 kg Trinatriumcitrat . 2$H_2O$ in 450 l siedendem demineralisiertem Wasser löst. Die Temperatur der Lösung wird sodann langsam bis auf etwa 70 - 80 °C erniedrigt. Anschließend wird die Lösung mittels einer Pumpe kontinuierlich auf einen Walzentrockner und gemäß Herstellungsbeispiel 1 zur Trockne gebracht. Das Gewicht des Endprodukts, berechnet als wasserfreie Substanz, beträgt 907 kg.

**Herstellungsbeispiel 5**: $KNaH(C_6H_5O_7)$ als Hydratgemisch

Die Herstellung von $KNaH(C_6H_5O_7)$ erfolgt, indem man 960,6 kg Citronensäure unter Rühren in der 0.5 bis 1fachen Gewichtsmenge demineralisiertem Wasser, bezogen auf das Gewicht der eingesetzten Citronensäure, bei 90 °C löst, anschließend 290 kg wasserfreies Natriumcarbonat und 345 kg wasserfreies Kaliumcarbonat zufügt und jeweils die homogene heiße Lösung der Schnelltrocknung, wie im Herstellungsbeispiel 1 angegeben, unterwirft. Das Gewicht des Endprodukts, berechnet als wasserfreie Substanz, beträgt 1260 kg.

Anstatt Natriumcarbonat, wie beschrieben, kann man 420 kg wasserfreies Natriumbicarbonat oder 200 kg wasserfreies Natriumhydroxid einsetzen. Anstatt Kaliumcarbonat, wie beschrieben, kann man 500 kg wasserfreies Kaliumbicarbonat oder 280 kg wasserfreies Kaliumhydroxid einsetzen.

Es hat sich überraschenderweise gezeigt daß die erfindungsgemäßen Alkalimetallhydrogencitrate in Form von Hydratgemisch sich beispielsweise bei der Bekämpfung von arteriosklerotisch bedingten Kalkablagerungen in Blutgefäßen besonders gut eignen.

Die zur Anwendung kommenden Zusammensetzungen bzw. Verbindungen können zusammen mit ein oder mehreren Zusatzstoffen als Tablette, Dragee, Kapsel, Pille, Granulat, Emulsion, Brausetablette oder Lösung appliziert werden, wobei deren Wirkstoffgehalt einem Bruchteil oder einem Vielfachen einer Einzeldosis entspricht. Die Dosierungseinheiten können zum Beispiel l, 2, 3 oder 4 Einzeldosen oder 1/2, 1/3 oder 1/4 einer Einzeldosis enthalten.

**Formulierungsbeispiel**:

Eine Einzeldosis kann beispielsweise als Brausetablette bzw. als Brausegranulat 2002 mg Kaliumhydrogencarbonat und 1280,64 mg Citronensäure (entsprechend 20 mmol Kalium und 6,67 mmol Citrat bzw. 20 Basen-Milliäquivalente) und als Zusatzstoffe einen Aromastoff, Saccharin und Dimeticon sowie einen Sprengstoffpartner, wie eine physiologische organische Säure, beispielsweise Citronensäure, Weinsäure oder Bernsteinsäure, enthalten. Zum Formulierungsbeispiel würde z.B. als Sprengstoffpartner eine zusätzliche Menge von 1242,40 mg Citronensäure geeignet sein.

In einer weiteren Ausführungsform können nicht-toxische, inerte pharmazeutische geeignete Zusatzstoffe als feste, halbfeste oder flüssige Verdünnungsmittel, Füllstoffe und Formulierungshilfsmittel zusammen mit den Alkalimetallhydrogencitraten appliziert werden. Als Zusatzstoffe eignen sich vorzugsweise

(a) Füll- und Streckmittel, zum Beispiel Stärke, Milchzucker, Rohrzucker und Glucose,

(b) Bindemittel, zum Beispiel Gelatine, Carboxymethylcellulose und Alginate,

(c) Feuchthaltemittel zum Beispiel Glycerin,

(d) Sprengmittel, zum Beispiel Agar-Agar, Calciumcarbonat und Natriumcarbonat,

(e) Lösungsverzögerer, zum Beispiel Paraffin,

(f) Resorptionsbeschleuniger, zum Beispiel quarternäre Ammoniumverbindungen,

(g) Netzmittel, zum Beispiel Glycerinmonostearat,

(h) Adsorptionsmittel, zum Beispiel Kaolin und Bentonit und (i) Gleitmittel, zum Beispiel Talkum, Calcium- und Magnesiumstearat,

(j) geruchs- oder geschmackverbesserte Zusätze

(k) Karminativum z.B. Dimeticon

oder Gemische der unter (a) bis (k) aufgeführten Stoffe.

Als Trägerstoff für Lösungen und Emulsionen eignen sich vorzugsweise Wasser, Äthylalkohol und Öle, zum Beispiel Olivenöl, Maiskeimöl und Erdnußöl oder Gemische dieser Stoffe.

Es hat sich gezeigt, daß überraschenderweise durch die erfindungsgemäße Verwendung das Entstehen von Ablagerungen in Blutgefäßen verhindert wird und deren Auflösung in Blutgefäßen erfolgt. Dabei wird, wie in den Vergleichsversuchen gezeigt wird (s.später), bereits frühzeitig in die Genese der Ablagerungsbildung eingegriffen. Das bedeutet, daß das erfindungsgemäße Produkt schon die beginnende Mineralisation durch Einlagerung von Calciumverbindungen, i.e. Calciumphosphate, in die Media der Gefäße verhindert. Damit ist erstmals und entgegen der Meinung des Standes der Technik (s.Einleitung) eine Möglichkeit eröffnet worden, bereits frühzeitig vorbeugende Maßnahmen zur Bekämpfung der Ablagerungen durchzuführen.

Die erfindungsgemäße Verwendung wirkt jedoch auch auf den Abbau der Ablagerungen, so daß, wenn bereits eine Mineralisation in den Gefäßen fortgeschritten ist, beispielsweise durch Einlagerung von Calciumphosphaten als Vorstufen von Hydroxylapatit, eine wirkungsvolle Therapie ermöglicht wird.

Erfindungsgemäß werden besonders gute Therapieergebnisse erhalten, wenn als Alkalicitrate $KNaH(C_6H_5O_7)$, $K_6Na_6H_3(C_6H_5O_7)_5$, $K_2H(C_6H_5O_7)$, $Na_3C_6H_5O_7$ oder $K_3C_6H_5O_7$ auch in Form eines Hydratgemisches mit 1 bis 4 Wassermolekülen verwendet werden.

Die Erfindungsgemäße Verwendung erfolgt vorzugsweise oral.

Die Erfindung sei im folgenden näher unter Bezugnahme auf die beigefügten Abbildungen erläutert.

In den Abbildungen zeigen:

**Abb. (1A)**  einen Querschnitt eines Aortaabschnitts von unbehandelten Ratten (Versuchsgruppe"Kontrolle")

**Abb. (1B)**  einen Querschnitt eines Aortaabschnitts von mit $K_6Na_6H_3(C_6H_5O_7)_5$ behandelten Ratten (Versuchsgruppe "KNC")

**Abb. (2A)**  Schichtdicke der Ablagerungen ($\mu$m) der KC-, KNC-, NC-Versuchsgruppen

**Abb. (2B)**  Ausmaß der Ablagerungsfläche in Aortasegmenten der KC-, KNC-, NC-Versuchsgruppen

**Vergleichsversuche:**

Die Vergleichsversuche wurden mit Ratten (unter Beachtung des deutschen Tierschutzgesetzes) durchgeführt, da Vergleichsversuche bei Menschen aufgrund der oft langsam fortschreitenden Ablagerungsbildungen sehr zeitaufwendig gewesen wären. Außerdem wären bei den einzelnen Versuchsgruppen Versuchspersonen mit Ablagerungsbildungen in zeitlich identischem Erkrankungsstadium nötig gewesen, ein

EP 0 642 790 A2

Umstand, der nur unter Einsatz größten diagnostischen Aufwandes möglich wäre.

Daher wurden Vergleichsversuche mit Ratten durchgeführt, die im Ergebnis denen bei Menschen bez. der Identität der Ablagerungszusammensetzung und der Physiologie der Erkrankung entsprechen.

Methodik:

Pentobarbital betäubten Ratten (Sprague-Dawley-Ratten) wurde die Aorta aseptisch entnommen, in acht 5 mm große Segmente geschnitten und in sterile Diffusionskammern (Fa. Millipore GmbH, Eschborn), mit einem Durchmesser von 14 mm und einer Porengröße von 0,22 $\mu$m fixiert. Jeweils 2 Kammern wurden in die Bauchhöhle einer Ratte implantiert. Die Versuchsgruppen bestanden aus mindestens 7 Ratten.

Folgende wäßrige Citrat-Lösungen wurden oral appliziert:

| Gruppenbezeichnung | Lösungen |
|---|---|
| **KC** | $K_3 C_6 H_5 O_7 \times 1\ H_2 O$ |
| **KNC** | $K_6 Na_6 H_3 (C_6 H_5 O_7)_5 \times 2\ H_2 O$ |
| **NC** | $Na_3 C_6 H_5 O_7 \times 2\ H_2 O$ |
| **Kontrolle** | demineralisiertes Wasser |

Jedem Versuchstier wurden pro Tag 2,1 mmol Citrat oral in zwei gleichen Dosen appliziert, nach 22 Tagen die beiden Diffusionskammern entnommen und die Aorta-Abschnitte licht-, elektronenmikroskopisch, röntgenspektrographisch sowie elementar-analytisch untersucht.

Zur histologischen Untersuchung wurden die Gewebeabschnitte in 8% Formalin fixiert, die Aortaabschnitte für die lichtmikroskopischen Untersuchung nach Kossa mit Hämatoxylin und Eosin gefärbt sowie diejenigen zur elektronenmikroskopischen Untersuchung in 2,5% Glutaraldehyd und anschließend in 1% Osmiumtetroxyd fixiert. Die Bestimmung der maximalen Ablagerungsschichtdicke erfolgte mittels Okularmikrometer und die Flächenausdehnung der Ablagerungsschicht der jeweiligen Aortasegmente wurden nach bekannten Methoden ermittelt.

**Histologische Ergebnisse**:

Bei histologisch-lichtmikroskopischer Untersuchung der Querschnitte der Aortaabschnitte sind in der Kontrolle eine ausgeprägte Ablagerungsbildung erkennbar [Abb.(1A)], wohingegen die mit KNC behandelte Gruppe [Abb. (1B)] eine gleichmäßig gestaltete (ohne besondere Erhebungen), stark verringerte Plaqueschicht aufweist. Auch die mit NC und KC behandelte Gruppen zeigen eine gleichmäßig verteilte ohne besondere Erhebungen gestaltete Ablagerungsschicht.

Bei der quantitativen Überprüfung werden bei der mit NC behandelten Gruppe eine um mehr als den Faktor 2 erfolgte Abnahme der Ablagerungsschichtdicke im Vergleich zu der Kontrolle von 55 $\mu$m auf ca. 26 $\mu$m festgestellt [Abb.(2A)]. Bei der mit KC applizierten Gruppe verringert sich die Ablagerungsdicke auf 33 $\mu$m.

Die weitaus am größten hervorgerufene Minimierung der Ablagerungsschichtdicke zeigt die mit KNC behandelte Gruppe auf. Bei ihr verringert sich die Ablagerungsdicke um den Faktor 2,4 auf weniger als 55 % im Gegensatz zur Kontrolle.

Da bei den Ablagerungen das Ulcerieren arteriosklerotischer Beete mit dem Dickerwerden der Konkrementschicht steigt - besonders in der menschlichen Aorta -, erweist sich somit die Behandlung mit KC, NC, insbesondere aber mit KNC, als sehr erfolgreich.

Die Untersuchung bez. der Flächenausdehnung der Ablagerungen ergibt sowohl bei der KC-Gruppe als auch bei der KNC-Gruppe eine ausgeprägte Minimierung im Vergleich zur Kontrolle um 41 % bzw 54 %.

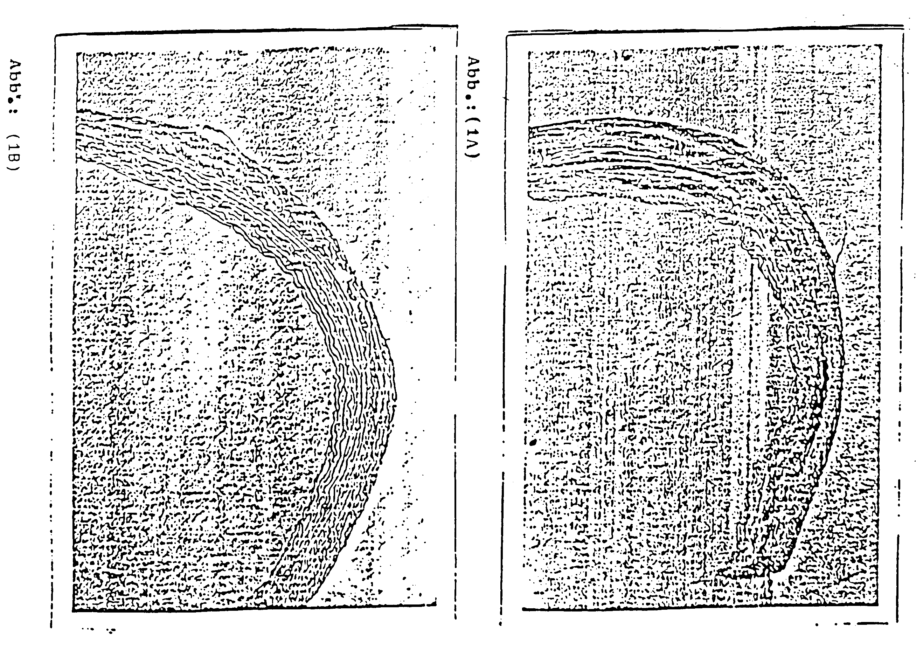

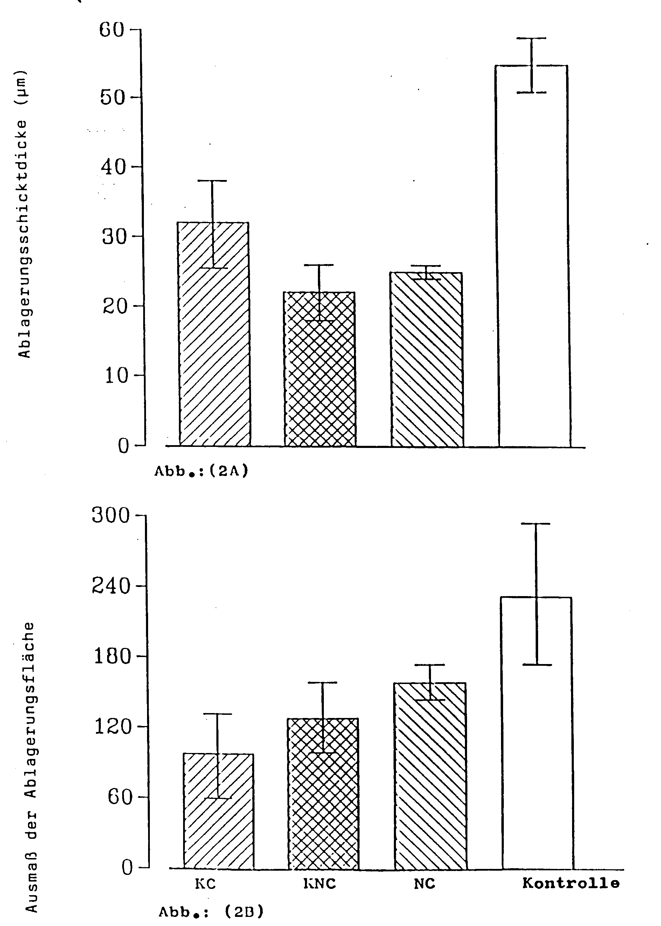

Die Ergebnisse der Abb. 2B zeigen die Wirkung der eingesetzten erfindungsgemäßen Substanzen gegenüber den Kontrollen.

Um einen Näherungswert bez. der relativen Volumina der Ablagerungen zu errechnen, erfolgt eine Multiplikation der Werte bez. der Dicke [Abb. (2A)] mit denen der Flächenausdehnung [Abb. (2B)]. Da beide Gruppen gleichmäßig ausgeprägte, ohne besondere Erhebungen gestaltete Ablagerungsschichten aufweisen (siehe oben), sind die berechneten relativen Volumina vergleichbar.

6

|  | KC | KNC | NC | Kontrolle |
|---|---|---|---|---|
| Dicke ($\mu$m) | 33 | 23 | 26 | 55 |
| Fläche | 98 | 131 | 159 | 240 |
| relative Volumina | 3234 | 3013 | 4134 | 13200 |
| % | 25 | 23 | 31 | 100 |

Hierbei zeigen die Ergebnisse von KNC im Vergleich zu denen der Kontrolle, daß sowohl bez. der Dicke als auch in Hinsicht auf die relativen Volumina der Ablagerungen die Werte der KNC-Gruppe am niedrigsten liegen.

In Tabelle (1) befinden sich die Werte bez. der Konzentrationen von Calcium bei allen Gruppen im Vergleich zur Kontrolle im Normbereich. In Hinsicht auf die von Magnesium ist auffallend, daß die KNC-Gruppe den höchsten Magnesiumanstieg aufweist. Da Magnesium ein Antagonist von Calcium ist, könnte möglicherweise die verstärkte Anwesenheit von Magnesium die verringerte Ablagerungsbildung gerade in der KNC-Gruppe erklären, welche auch die geringste Calciumphosphat-Einlagerung aufweist.

In Tabelle (1) ist der Calcium/Phosphor-Quotient in der Kontrolle kleiner als 1,5. Da allgemein anerkannt ist, daß das Calcium/Phosphor-Verhältnis von Hydroxylapatit jedoch größer als 1,5 ist, muß von der Anwesenheit von Vorläufern des Hydroxylapatits in der Kontrolle ausgegangen werden.

Weil gleichfalls die KC-, KNC- und NC-Versuchsgruppen einen Calcium-Phosphor-Quotienten von weniger als 1,5 demonstrieren, bedeutet das, daß die Applikation von Citrat, insbesondere die von KNC, gerade die "Reifung" von Calciumphosphat (als Vorläufer) zu Hydroxylapatit in den Ablagerungen der Media der Blutgefäße stark einschränkt und die Einlagerung von Vorläufern des Hydroxylapatits wirkungsvoll verhindert.

Diese Ergebnisse demonstrieren, daß erstens in der Kontrolle die Ablagerungen durch Calciumphosphateinlagerung entstehen - und auch den physiologischen Gegebenheiten im menschlichen Organismus vergleichbar sind -, und daß zweitens gerade durch die Einnahme von Citraten, e.g. Alkalicitraten wie KNC, wirkungsvoll die Ablagerungsbildung eingeschränkt und sogar bei dauernder Applikation verhindert wird sowie ein Abbau stattfindet.

Diese Möglichkeit stellt erstmals und entgegen der Meinung des Stands der Technik (siehe Einleitung) eine wirkungsvolle sowie therapeutische vorbeugende Maßnahme in der Bekämpfung von Ablagerungen bei arteriosklerotischen Erkrankungen dar.

Diese Ergebnisse werden sowohl von elektronenmikroskopischen und röntgenspektrographischen Untersuchungen bestätigt, welche das Vorliegen von amorphen Calcium enthaltenden Strukturen, und nicht die Anwesenheit von kristallinem Hydroxylapatit, offenbaren als auch von Mikroelementaranalysen der Ablagerungsschichten bekräftigt, die einen Calcium-Phosphor-Quotienten von kleiner als 1,25 angeben.

Die klinisch-chemische Untersuchung [Tabellen (2A)] zeigt, daß der arteriell-venöse pH-Wert, die Konzentrationen an Gesamt-$CO_2$, Citrat, Magnesium und Phosphor im Blut durch die Applikation von KNC, KC und NC nicht verändert werden und sich im Normbereich im Vergleich zur Kontrolle befinden. Das bedeutet, daß die Verstoffwechslung erhöhter Gaben von KNC, KC oder NC im Organismus nicht zu einer Veränderung der Serumzusammensetzung führt und daß daher die Nierenfiltration aufgrund der im Normbereich befindlichen Fraktionen der ultrafiltrierbaren und ionablen Fraktion des Serums nicht beeinträchtigt wird.

Der Umstand, daß Alkalicitrate ohne Nebenwirkungen im Gegensatz zu bekannten o.g. Medikamente auf die Nierentätigkeit ist, ergibt sich auch aus der Bestimmung der Urinzusammensetzung [Tabelle (2B)). Die Konzentrationen von beispielsweise cAMP unterschieden sich nur unwesentlich von denen der Kontrolle.

Diese Versuche zeigen eindrucksvoll, daß die Applikationen mit KNC und weiteren Citraten aufgrund der Identität der Konzentrationen der Stoffwechselprodukte sowohl im Harn als auch im Blut zu einer ausgezeichneten Verträglichkeit der Substanzen im Organismus als auch zu keinen nennenswerten Nebenwirkungen führen.

Um die Wirkung der Applikationen von KNC, NC und KC auf den Hormonhaushalt zu überprüfen, wurde die Konzentration von PTH im Blut analysiert. Gleichfalls finden sich hier keine Unterschiede im PTH-Level zwischen der Kontrolle und KNC-Zugabe, so daß trotz KNC Zugabe ein ausgeglichenes, von der Kontrolle nicht unterscheidbares Hormongleichgewicht vorliegt.

Die Untersuchungen demonstrieren, daß KNC weder die Nierentätigkeit aufgrund der Konstanz in den Blutgasanalysewerten und im mineralischen Stoffwechselhaushalt beeinflußt noch in den Hormonhaushalt störend eingreift. KNC und die übrigen Citrate sind somit gut und schnell im Organismus abbaubar. Da KNC im Organismus zu Citrat dissoziiert, sollte es auch in Kombination mit weiteren Wirkstoffen, solange diese nicht mit dem körpereigenen Citrat in Wechselwirkung treten, keine Kontraindikation vorweisen.

Da zur Therapie eine körpereigene Substanz, i.e. Citronensäure bzw. deren Salz, appliziert wird, und da in den Vergleichsversuchen bei der Einnahme von KNC keine Änderungen im Stoffwechselhaushalt zu erkennen sind, ist ebenso eine Langzeiteinnahme möglich, wie es gerade die frühzeitig beginnende, vorbeugende Behandlung mit beispielsweise KNC gegen die Einlagerung und die Reifung der Calciumphosphat-Vorläufer zu Hydroxylapatit erfordert.

Auch sind die erforderlichen Ausgangssubstanzen, wie Citronensäure, Trikaliumcitrat, Trinatriumcitrat, Kalium- oder Natriumcarbonate ggf. -bicarbonate oder -hydroxide, zur Herstellung des erfindungsgemäßen jeweiligen Alkalicitrats chemische Massenprodukte und somit preiswerte Grundstoffe, so daß auch das Erfordernis der kostengünstigen Vorbeugung und Therapie zur Verhinderung und Auflösung von Ablagerungen bei arteriosklerotischen Erkrankungen erfüllt ist.

**Versuchsgruppen**

| | KC | KNC | NC | Kontrolle |
|---|---|---|---|---|
| **Rattenanzahl** | | | | |
| | 9 | 10 | 7 | 11 |
| **Konzentration [mmol/g]** | | | | |
| Calcium | 5,09 | 4,93 | 4,20 | 4,77 |
| | (3,15-10,81) | (3,40-7,16) | (2,61-10,90) | (2,83-6,90) |
| Magnesium | 0,28 | 0,30, | 0,28 | 0,20 |
| | (0,20-0,43)[2] | (0,22-0,39)[1] | (0,20-0,33)[1] | (0,13-0,35) |
| Phosphor | 7,66 | 6,80 | 6,35 | 4,47 |
| | (2,97-17,74) | (2,09-10,51)[1] | (3,34-14,60) | (3,05-6,03) |
| Calcium/ Phosphor- Quotient | 0,70 | 0,75 | 0,66 | 1,16 |
| | (0,61-1,06)[1] | (0,61-1,24)[1] | (0,23-1,02) | (0,64-2,07) |

Tabelle 1: Mineralstoffgehalt und Calcium/Phosphor-Quotient in Aortasegmenten;
die Werte sind gemittelt; die Werte in Klammern stellen
die Bereiche der gemessenen Werte dar;
[1]: p kleiner 0,017; [2]: p kleiner 0,01 bezogen auf Kontrolle

EP 0 642 790 A2

EP 0 642 790 A2

Versuchsgruppen

----------------------------------------------------------------

| | KC | KNC | NC | Kontrolle |
|---|---|---|---|---|

----------------------------------------------------------------

Rattenanzahl pro Gruppe

| | 9 | 10 | 7 | 11 |
|---|---|---|---|---|

----------------------------------------------------------------

**Blut**

| | KC | KNC | NC | Kontrolle |
|---|---|---|---|---|
| pH | 7,39 (7,31-7,45) | 7,39 (7,34-7,43) | 7,43 (7,35-7,49) | 7,39 (7,39-7,44) |
| Ges.CO2 [mmol/g] | 45,0 (39,2-50,8) | 40,9 (36,9-45,1) | 40,9 (29,6-51,8) | 42,3 (36,3-48,0) |
| Citrat [μmol/l] | 163 (96-248) | 164 (55-244) | 171 (140-212) | 156 (72-193) |
| Kreatinin [μmol/l] | 31,8 (25,6-39,8) | 30,9 (15,9-35,4)[2] | 31,8 (30,1-33,6)[1] | 37,1 (28,3-44,2) |
| Ges.Protein [g/l] | 51,0 (48,3-53,3)[2] | 50,7 (47,5-57,0)[2] | 48,3 (44,8-55,3)[2] | 54,1 (50,3-57,8) |
| Calcium [mmol/l] | 2,27 (2,20-2,43)[1] | 2,27 (2,22-2,33)[1] | 2,20 (2,04-2,30)[3] | 2,36 (2,26-2,57) |
| Calcium/ Protein [μmol/g] | 44 (42-46) | 45 (40-47) | 46 (42-50) | 44 (40-51) |
| PTH | 200-468 | 200-553 | 200-415 | 280-481 |

----------------------------------------------------------------

Tabelle 2A: Klinisch-chemische Untersuchung der Fraktionen im Blut; die Werte sind gemittelt; die Werte in Klammern stellen die Bereiche der gemessenen Werte dar; [1]: p kleiner 0,017; [2]: p kleiner 0,01; [3]: p kleiner 0,001 bezogen auf Kontrolle

| Versuchsgruppen | KC | KNC | NC | Kontrolle |
|---|---|---|---|---|
| Rattenanzahl pro Gruppe | 9 | 10 | 7 | 11 |
| **Urin** **Werte pro Kreatinin** | | | | |
| cAMP [nmol/mg] | 12,9 (9,9-16,0) | 11,6 (9,0-15,9) | 13,5 (12,1-14,2) | 13,5 (10,3-15,8) |
| Magnesium [µmol/mg] | 3,4 (1,8-9,1)[3] | 3,7 (1,4-8,0)[3] | 4,0 (2,6-8,0)[3] | 20,8 (12,5-28,1) |
| Citrat [µmol/g] | 22,0 (11,3-25,0)[2] | 20,0 (13,2-30,3)[1] | 19,7 (15,8-30,4)[2] | 15,4 (7,1-18,7) |

Tabelle 2B: Klinisch-chemische Untersuchung der Fraktionen im Urin; die Werte sind gemittelt; die Werte in Klammern stellen die Bereiche der gemessenen Werte dar; [1]: p kleiner 0,017; [2]: p kleiner 0,01; [3]: p kleiner 0,001 bezogen auf Kontrolle

## Patentansprüche

1. Verwendung mindestens einer Alkalimetallhydrogencitratzusammensetzung oder -Verbindung oder eines Hydrats davon mit der Zusammensetzung Kaliumnatriumhydrogencitrat im Molverhältnis

K:Na:H:($C_6H_5O_7$) w:x:y:z

worin w für eine ganze Zahl von 0 bis 15; x für eine ganze Zahl von 0 bis 15; y für eine ganze Zahl von 0 bis 3; z für eine ganze Zahl von 1 bis 5 stehen; wobei

w + x + y = 3; 6; 9; 12 oder 15 bei entsprechend

z = 1; 2; 3; 4 oder 5 und w + x = 1 bis 15 sind, zur Prophylaxe und Auflösung von Kalkablagerungen in Blutgefäßen.

2. Verwendung nach Anspruch 1 zur Prophylaxe und Behandlung bei Arteriosklerose.

3. Verwendung nach Anspruch 1 oder 2, **dadurch gekennzeichnet**, daß das Alkalimetallcitrat $KNaH(C_6H_5O_7)$, $K_6Na_6H_3(C_6H_5O_7)_5$, $K_2H(C_6H_5O_7)$, $Na_3(C_6H_5O_7)$ oder $K_3(C_6H_5O_7)$ bzw. eines Hydrats davon ist.

4. Verwendung nach Anspruch 1 oder 2, wobei das Alkalimetallhydrogencitrat in Form einer Mischung aus Kaliumhydrogencarbonat und/oder Natriumhydrogencarbonat und Citronensäure bereitgestellt wird.

5. Verwendung nach Anspruch 4, wobei die Citronensäure im Überschuß verwendet wird.

6. Kalium-Natrium-Hydrogencitrat der Formel

$KNaH(C_6H_5O_7)$

oder ein Hydrat davon, insbesondere mit 1 bis 4 Wassermolekülen.

7. Verfahren zur Herstellung der Verbindung gemäß Anspruch 6, **dadurch gekennzeichnet**, daß man entweder

a) Trinatriumcitrat, Trikaliumcitrat und Citronensäure im Molverhältnis 1:1:1 gemeinsam in der 3 bis 5fachen Gewichtsmenge in siedendem demineralisiertem Wasser, bezogen auf das Gewicht der Citronensäure, auflöst, die Temperatur der Lösung 60 °C nicht unterschreiten läßt oder

b) Citronensäure unter Rühren in der 0,5 bis 1fachen Gewichtsmenge deminieralisiertem Wasser, bezogen auf das Gewicht der eingesetzten Citronensäure, bei 90 °C löst anschließend Natriumcarbonat sowie Kaliumcarbonat in solchen Mengen zufügt, daß sich ein Molverhältnis von Natriumcarbonat:Kaliumcarbonat:Citronensäure von 1:1:2 ergibt oder

c) Citronensäure unter Rühren in der 0,5 bis 1fachen Gewichtsmenge demineralisiertem Wasser, bezogen auf das Gewicht der eingesetzten Citronensäure, bei 90 °C löst, anschließend Natriumhydroxid oder Kaliumbicarbonat sowie Kaliumhydroxid oder Kaliumbicarbonat in solchen Mengen zufügt, daß sich ein Molverhältnis von Natriumhydroxid oder Natriumbicarbonat:Kaliumhydroxid oder Kaliumbicarbonat:Citronensäure von 1:1:1 ergibt

und jeweils die homogene heiße Lösung einer Schnelltrocknung unterwirft.

8. Verfahren nach Anspruch 7, **dadurch gekennzeichnet**, daß man die jeweiligen Ausgangssubstanzen im Falle der Variante a) in der 3,7fachen, und im Falle der Variante b) und c) in der 0,63fachen Gewichtsmenge demineralisiertem Wasser, bezogen auf das Gewicht der eingesetzten Citronensäure, auflöst.

9. Pharmazeutisches Mittel, enthaltend die Verbindung nach Anspruch 6, ggf. in Kombination mit üblichen Trägern und/oder Zusatzstoffen.

Abb.: (1A)

Abb.: (1B)

Abb.:(2A)

Abb.: (2B)